# EUROPÄISCHE PATENTSCHRIFT

(11) **EP 1 998 704 B1**
(45) Veröffentlichungstag und Bekanntmachung des Hinweises auf die Patenterteilung: **07.12.2011**
(21) Anmeldenummer: 07723830.1
(22) Anmeldetag: 30.03.2007
(51) Int. Cl.: A61C 1/08, A61C 8/00

(54) **ANORDNUNG ZUR INSERTION VON IMPLANTATEN**
SYSTEM FOR INSERTION OF IMPLANTS
SYSTEME D'INSERTION D'IMPLANTS

(30) Priorität: 30.03.2006 DE 102006015215; 21.11.2006 DE 102006055212
(43) Veröffentlichungstag der Anmeldung: 10.12.2008
(73) Patentinhaber: FRIADENT GmbH, 68229 Mannheim (DE)
(72) Erfinder: WOLF, Dietrich, 73447 Oberkochen (DE); BERGNER, Norbert, 68229 Mannheim (DE)
(74) Vertreter: Schmitt, Meinrad
(86) Internationale Anmeldenummer: PCT/EP2007/002888
(87) Internationale Veröffentlichungsnummer: WO 2007/112977

(56) Entgegenhaltungen:
- EP-A- 1 759 658
- EP-A2- 0 328 911
- WO-A-97/49351
- WO-A-99/26540
- US-A1- 2005 170 311

## Beschreibung

Die Erfindung bezieht sich auf eine Anordnung zur Insertion von Implantaten, insbesondere Dentalimplantaten, gemäß den im Oberbegriff des Patentanspruchs 1 angegebenen Merkmalen.

Aus der US 2005/0170311 A1 ist eine derartige Anordnung bekannt, welche eine als Schablone ausgebildete Schiene zur Positionierung des oder der Implantate enthält. Die Schiene wird aufgrund von Daten hergestellt, welche mittels eines Computer-Tomographen (CT), eines Röntgengerätes oder einer sonstigen bilderzeugenden Einrichtung des Knochens, insbesondere des Kieferknochens, erzeugt werden, um die erforderliche Position des Implantats unter Berücksichtigung der anatomischen, chirurgischen und auch der ästhetischen Gegebenheiten planen und definiert vorgeben zu können. Die Schiene ist auf die jeweilige Situation und Gegebenheiten angepasst und enthält wenigstens eine durchgehende Bohrung zur Aufnahme einer Führungshülse mit einer Durchgangsbohrung, gemäß welcher die exakte Ausrichtung und Positionierung der Bohrung für das. Implantat vorgebbar ist. Ferner ist eine in die Führungshülse der Schiene einsetzbare Bohrhülse mit einer Durchgangsbohrung vorgesehen, deren Innendurchmesser auf den Außendurchmesser des jeweiligen Bohrers zum Einbringen der Bohrung in den Knochen abgestimmt ist. Der Bohrer enthält einen Ringbund oder Anschlag, welcher auf der freien Oberkante der Bohrhülse zur Anlage bringbar ist, um somit die Tiefe der Bohrung im Knochen vorzugeben. Ferner wird die Schiene zum Einbringen der Implantate genutzt, mit welchen besondere Befestigungskörper mittels Schrauben verbunden sind. Die Befestigungskörper enthalten einen dem jeweiligen Implantat zugewandten zylindrischen Bereich, dessen Außendurchmesser auf den Innendurchmesser der in der Schiene festgelegten Führungshülse abgestimmt ist. Der Befestigungskörper enthält einen dem Implantat abgewandten Bereich mit beispielsweise als Sechskant ausgebildeten Eingriffsflächen für ein Eindrehwerkzeug. In axialer Richtung zwischen den beiden genannten Bereichen besitzt der Befestigungskörper einen Flansch, welcher zur Begrenzung der Einbringtiefe des Implantats an der freien Oberkante des Führungszylinders zur Anlage gebracht wird.

Der Flansch weist einen erheblich größeren Durchmesser als der zylindrische Bereich auf, so dass bei engen Platzverhältnissen Probleme entstehen können. Der Befestigungskörper weist eine nicht unerhebliche axiale Länge auf, wodurch die Handhabung beim Eindrehen des Implantats im Hinblick auf die engen Platzverhältnisse in der Mundhöhle nicht unerheblich erschwert wird. Da bekanntlich lmplantatsysteme Implantate mit unterschiedlichen Durchmessern aufweisen, ist eine erhebliche Anzahl derartiger Befestigungskörper erforderlich. Auch ist das Einbringen und die exakte Positionierung von sogenannten Miniaturimplantaten oder Miniimplantaten mit Außendurchmessern in der Größenordnung von 1 mm und kleiner nicht ohne weiteres möglich, welche überwiegend einteilig ausgebildet sind und transgingival eine minimal invasive Insertion ermöglichen. Beim Einbringen der Implantate in das mittels der genannten Anordnung aufbereitete Implantatbett ergeben sich in der Praxis, vor allem bei Miniimplantaten, Probleme dahingehend, dass die Einbringtiefe des Implantats vom Operateur nur sehr schwer kontrollierbar ist und ferner im Hinblick auf den durch den Befestigungskörper bedingten recht großen Abstand des Eindrehwerkzeugs zum Implantat unerwünschte Kippmomente oder Drehungen verursacht werden können, welche zu Fehlpositionierungen führen.

Ferner ist aus der WO 97/49 351 A eine Anordnung bekannt, enthaltend eine Bohrplatte, welche mittels eines Abstandshalters oberhalb des Knochens angeordnet wird, und eine Befestigungsplatte mit zylindrischen Ansätzen, welche in Bohrungen der Bohrplatte eingreifen. Mittels Stützschrauben werden die Bohrplatte und die Führungsplatte bezüglich des Knochens fixiert. Die genannten zylindrischen Ansätze enthalten Durchgangsbohrungen für Führungselemente mit radialen Flanschen, welche nach dem Fixieren und dem Zusammenbau der Bohrplatte und der Führungsplatte auf deren Oberfläche aufliegen. Die Bohrplatte enthält an ihrer dem Knochen zugewandten Unterseite eine gestufte Bohrung, in welche ein Abstandshalter teilweise eingreift. Der Abstandshalter ist mit einem Implantat verbunden, welches zuvor in den Knochen insertiert wurde, wobei kein Hinweis zur Vorgabe der exakten Einbringtiefe dieses Implantats entnehmbar ist. Der zweiteilige Aufbau der Anordnung aus der Bohrplatte und der Führungsplatte erfordert einen nicht unerheblichen Material- und Fertigungsaufwand.

Des Weiteren ist aus der WO 99/26 540 eine Anordnung mit einer Schablone bekannt, welche mehrere Master-Zylinder jeweils mit gleichen Außenabmessungen enthält. In diese Zylinder können Bohrhülsen eingesetzt werden, deren Außendurchmesser auf den Innendurchmesser der Master-Zylinder abgestimmt sind. Die Innendurchmesser der Bohrhülsen sind jedoch unterschiedlich entsprechend den Durchmessern der jeweils zum Einsatz gelangenden Bohrer und Implantate ausgebildet.

Hiervon ausgehend liegt der Erfindung die Aufgabe zugrunde, die aufgezeigten Nachteile zu vermeiden und die Anordnung dahingehend weiterzubilden, dass die exakte Einbringung des Implantats mit hoher Sicherheit vorgebbar ist, insbesondere zusätzlich zur exakten Positionierung der Bohrung. Die Anordnung soll bei einfacher und funktionssicherer Konstruktion eine problemlose und zuverlässige Handhabung beim Einbringen der Bohrung in den Knochen und / oder bei der Insertion des Implantats sicherstellen.

Die Lösung dieser Aufgabe erfolgt gemäß den im Patentanspruch 1 angegebenen Merkmalen.

Die erfindungsgemäße Anordnung zeichnet sich durch eine funktionsgerechte Ausbildung aus und ermöglicht problemlos das Einbringen des Implantats mit vorgebbarer definierter Einbringtiefe. Das Eindrehwerkzeug ist unmittelbar mit dem Implantat verbunden und besitzt einen Kopfteil, dessen Außenfläche zur Führung beim Eindrehen benutzt wird. Der Außendurchmesser des Kopfteils ist auf den Innendurchmesser der Innenfläche der in der Schiene vorgesehenen Ausnehmung und / oder Bohrung abgestimmt. In vorteilhafter Weise gelangen für Implantate mit unterschiedlichen Außendurchmessern jeweils gleiche und/oder in den Außenabmessungen übereinstimmende Eindrehwerkzeuge zum Einsatz. Da keine besonderen Befestigungskörper zwischen dem Eindrehwerkzeug und dem Implantat vorgesehen sind, entfällt der diesbezügliche Fertigungsaufwand und/oder Bereitstellungsaufwand. Die Schiene ist vorteilhaft zur Aufnahme einer Hülse zum Einbringen des Implantats ausgebildet, deren Innendurchmesser zumindest teilweise auf den Außendurchmesser des Implantats abgestimmt ist. Diese Hülse, nachfolgend Einbringhülse genannt, weist eine Anschlagfläche auf, und korrespondierend zu dieser besitzt, insbesondere das Eindrehwerkzeug, eine zweite Anschlagfläche derart, dass beim Eindrehen des Implantats und erfolgter Anlage der genannten Anschlagflächen ein weiteres Eindrehen unterbunden wird und somit die exakte Einbringtiefe des Implantats definiert vorgegeben ist. Die Anschlagflächen sind bevorzugt innerhalb der Ausnehmung und / oder Bohrung der zumindest einen Hülse vorgesehen. Die Anschlagflächen können als Stufen, Ringbunde oder dergleichen einerseits der Einbringhülse, Führungshülse oder der Bohrhülse oder der Schiene und andererseits des Bohrers oder des Eindrehwerkzeugs ausgebildet sein. Es ist von besonderer Bedeutung, dass vor allem bei Implantaten mit kleinen Außendurchmessern und insbesondere bei Miniaturimplantaten der Außendurchmesser des Kopfteils des Eindrehwerkzeugs wesentlich größer als der des Miniaturimplantats ist, wodurch in besonders vorteilhafter Weise eine exakte Einbringung und/oder Positionierung sichergestellt ist.

Besondere Ausgestaltungen und Weiterbildungen der Erfindung sind in den Unteransprüchen und der nachfolgenden Beschreibung eines besonderen Ausführungsbeispiels angegeben.

Die Erfindung wird nachfolgend anhand der Zeichnung näher erläutert, ohne dass insoweit eine Beschränkung erfolgt. Es zeigen:
- Fig. 1: eine Prinzipdarstellung des Kiefers mit der Schiene der erfindungsgemäßen Anordnung,
- Fig. 2, 3: Darstellungen gemäß Fig. 1 zusammen mit einem Bohrer in verschiedenen Axialpositionen,
- Fig. 4: eine Darstellung gemäß Fig. 1 nach dem Einbringen der Bohrung in den Kiefer,
- Fig. 5: eine Darstellung mit Eindrehwerkzeug und Implantat,
- Fig. 6: schematisch den Kiefer mit Implantat nach der Insertion,
- Fig. 7: eine schematische Darstellung des Kiefers mit den Verankerungskörpern von vier Imptantaten.

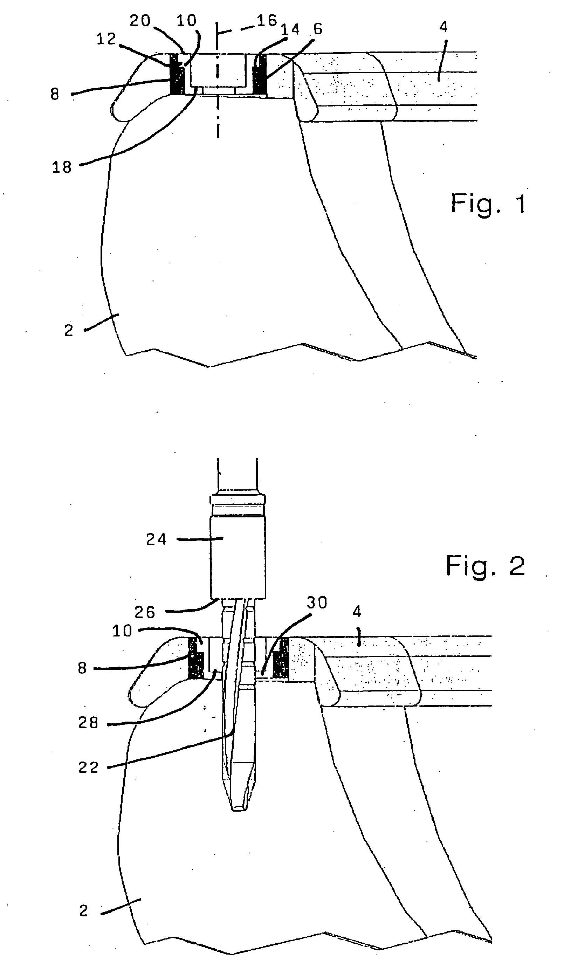

Fig. 1 zeigt schematisch einen Kiefer 2 einschließlich Schleimhaut und die darauf zur Positionierung der einzubringenden Bohrung und des Implantats vorgesehene Schiene 4. Die Schiene 4 enthält in einer Ausnehmung oder Bohrung 6 eine Führungshülse 8, welche zweckmäßig mit der Schiene 4, beispielsweise durch Kleben, fest verbunden ist. Alternativ kann die Führungshülse 8 lösbar bzw. einsetzbar in der Schiene 4 angeordnet sein. In der Führungshülse 8 ist eine lösbare Bohrhülse 10 angeordnet, wobei die Führungshülse 8, erste, zweckmäßig radial innenliegende Positionierungselemente 12 und ferner die Bohrhülse 10 radial außenliegende zweite Positionierungselemente 14 aufweisen. Die Positionierungselemente 12 und 14 sind insbesondere als miteinander korrespondierende Stufen ausgebildet und ermöglichen die axiale Positionierung der Bohrhülse 10 bezüglich einer Längsachse 16. Die Bohrhülse 10 enthält ferner, bevorzugt innenliegend, eine Anschlagfläche 18 zwecks Vorgabe der Bohrtiefe des hier nicht dargestellten Bohrers. Alternativ kann die gemäß Zeichnung obere Endfläche 20 als Anschlag für einen entsprechend ausgebildeten Bohrer dienen.

Des Weiteren kann im Rahmen der Erfindung die Führungshülse 8 integraler Bestandteil der Schiene 4 und / oder einteilig mit dieser ausgebildet sein. Ferner kann im Rahmen der Erfindung auch die Bohrhülse 10 integraler Bestandteil der Schiene 4 und / oder einteilig mit dieser ausgebildet sein, wobei die Schiene 4 aus einem Werkstoff besteht, welcher eine hinreichend große Festigkeit und / oder Härte besitzt, um eine Beschädigung durch den Bohrer zu vermeiden.

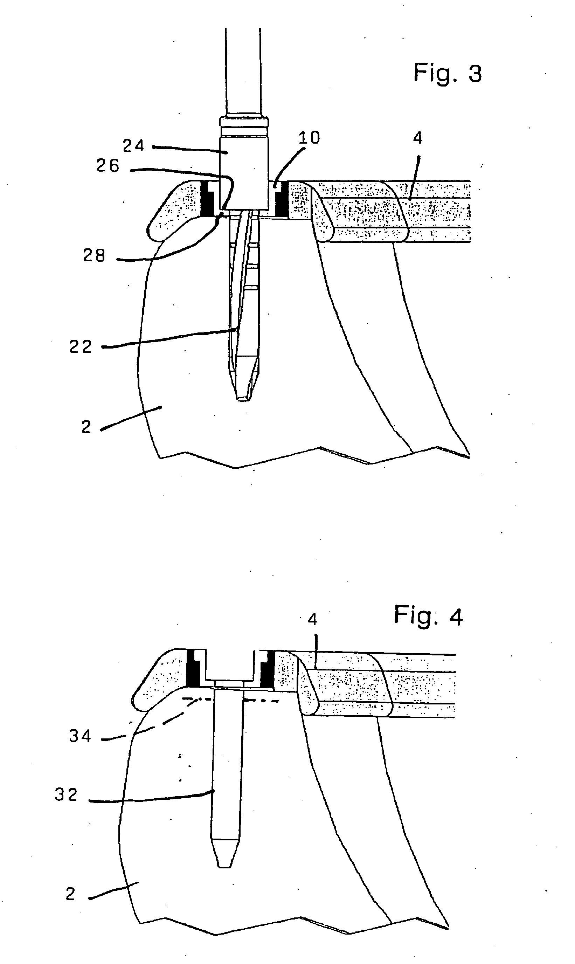

Fig. 2 und 3 zeigen zusätzlich den Bohrer 22, welcher in ein Bohrwerkzeug 24 eingesetzt ist. Das Bohrwerkzeug 24 enthält eine Anlagefläche 26, welche korrespondierend zur Anlagefläche 28 der Bohrhülse 10 ausgebildet ist. Im Übrigen sind der Außendurchmesser der Bohrwerkzeugs 24 und die Innenfläche der Bohrhülse 10 aufeinander abgestimmt, ebenso wie der Außendurchmesser des Bohrers 22 und der Innendurchmesser des Steges 30, welcher gemäß Zeichnung in Richtung zum Kiefer 2 unterhalb der Anlagefläche 18 in der Bohrhülse 10 angeordnet ist. Gemäß Fig. 3 liegt die untere Anlagefläche 26 des Bohrwerkzeugs 24 an der Anlagefläche 28 der Bohrhülse 10 an, und die vorgegebene Bohrtiefe des Bohrers 22 ist erreicht.

Gemäß Fig. 4 enthält nunmehr der Kiefer 2 die Einbringbohrung 32 bzw. das aufbereitete Implantatbett für das Implantat. Es sei festgehalten, dass die Schiene 4 auf den Kiefer 2 aufgesetzt wird, wobei mittels strichpunktierter Linie 34 der Grenzbereich zwischen der Schleimhaut und den Kieferknochen angedeutet ist. Ferner sei ausdrücklich festgehalten, dass die erfindungsgemäße Anordnung insbesondere für transgingivale Implantate zum Einsatz gelangt und minimal invasiv für eine sogenannte flapless Insertion unter Erreichung einer hohen Präzision ausgebildet ist.

Ferner können im Rahmen der Erfindung anstelle einer einzigen Bohrhülse auch mehrere Bohrhülsen eines Implantatsystems vorgesehen sein, deren Außengeometrien übereinstimmen und auf die Innengeometrie der Führungshülse bzw. der Ausnehmung der Schiene abgestimmt sind. Diese Bohrhülsen weisen vor allem im Bereich des erläuterten Steges Innendurchmesser auf, welche auf die unterschiedlichen Außendurchmesser der zum Einsatz gelangenden Bohrer abgestimmt sind. So kann beispielsweise mit einem vergleichsweise dünnen Bohrer zunächst eine Vorbohrung oder Pilotbohrung in den Kiefer eingebracht werden, um nachfolgend mittels eines oder mehrerer Bohrer, welche entsprechend vergrößerte Außendurchmesser aufweisen, das Implantatbett mit den erforderlichen Abmessungen aufzubereiten.

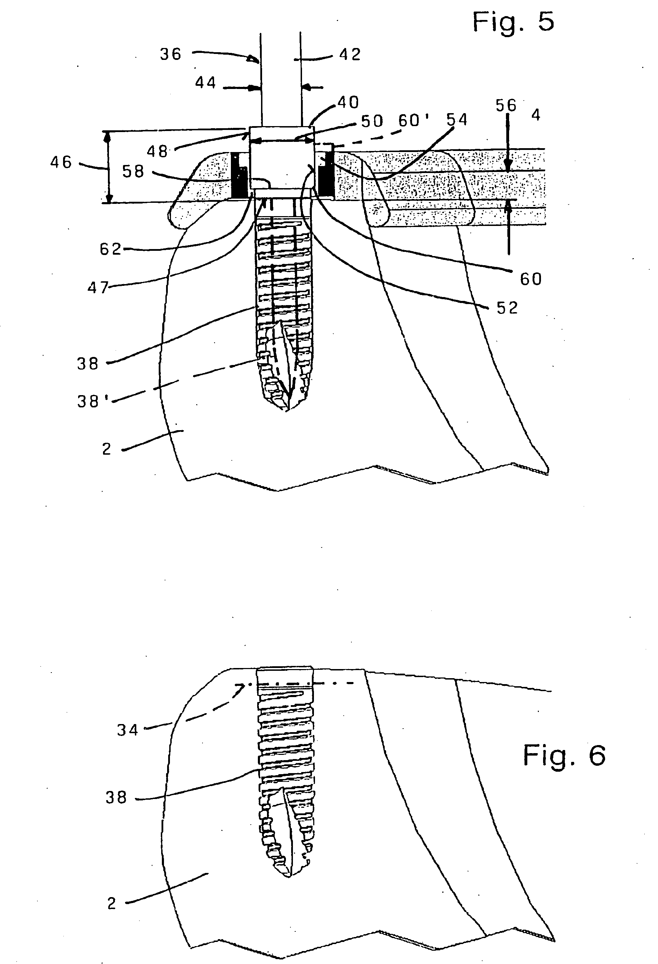

Fig. 5 zeigt ein Eindrehwerkzeug 36, mittels welchem das Implantat 38 in den Kiefer 2 insertiert ist. In bevorzugter Weise werden das Eindreh- oder Einbringwerkzeug 36 und das Implantat 38 vormontiert bereitgestellt, so dass insbesondere nach dem Einbringen der Bohrung in den Kiefer ohne weitere Hilfsmittel die Insertion des Implantats 38 durchgeführt werden kann. Das Eindreh- oder Einbringwerkzeug 36 enthält einen an das Implantat 38 anschließenden Kopfteil 40, direkt an diesen anschließend einen Schaft 42, dessen äußerer Durchmesser 44 bevorzugt wesentlich kleiner ist als der Außendurchmesser 50 des Kopfteils 40. Das Implantat 38 weist eine dem Kopfteil 40 zugeordnete axiale Stirnfläche 45 auf. Der Kopfteil 40 besitzt in axialer Richtung eine Länge 46 und eine Außenfläche 48 mit dem Durchmesser 50. Des Weiteren ist innerhalb der Schiene 4 eine axial durchgehende Ausnehmung 52 vorgesehen, an deren Innenfläche der Kopfteil 40 anliegt und/oder beim Eindrehen geführt wird, wobei die Innenfläche der Ausnehmung 52 die Führungsfläche bildet. Der Außendurchmesser 50 des Kopfteils 40 ist zumindest näherungsweise gleich groß wie der Innendurchmesser der Ausnehmung 52. die Länge 46 des Kopfteils 40 ist zumindest gleich groß wie die Dicke 56 der Schiene 4 im Insertionsbereich. In bevorzugter Weise ist die Länge 46 des Kopfteils 40 um einen vorgegebenen Faktor größer als die Dicke 56, wobei der Faktor maximal den Wert 2, bevorzugt maximal den Wert 1,6 und insbesondere 1,4 aufweist. Des Weiteren ist die gesamte axiale Länge des Einbringwerkzeugs 36 vergleichweise kurz vorgegeben, wobei die Länge vorteilhaft maximal um den Faktor 3, insbesondere maximal um den Faktor 2,5, größer als die Länge 46 vorgegeben ist. Erfindungsgemäß liegt der zylindrische Kopfteil 40 mit einer axialen Stirnfläche 47 unmittelbar an der zugeordneten Stirnfläche 45 des Implantates 38 an. Aufgrund der derart vorgegebenen recht geringen Länge des Einbringwerkzeugs 36 werden Hebel- oder Kippbewegungen des Einbringwerkzeugs 36 beim Einbringen des Implantats 38 in den Kiefer 2 gering gehalten und weitgehend vermieden, wodurch eine exakte Positionierung des Implantats 38 erleichtert wird. Des Weiteren wird die Handhabung beim Einbringen des Implantats aufgrund der kleinen Abmessungen des Einbringwerkzeugs verbessert.

Wie dargestellt, befindet sich die Ausnehmung und / oder Führungsfläche 52 in der Einbringhülse 54. In einer besonderen Ausgestaltung der Erfindung können das Einbringen der Bohrung und ferner die insoweit erläuterten Hülsen entfallen und das Implantat, insbesondere ein Miniaturimplantat, direkt mittels eines bevorzugt selbstschneidenden Gewindes insertiert werden. Ferner können das Eindrehwerkzeug 36 und das Implantat 38 vor der Insertion vom Operateur in geeigneter Weise und / oder mittels geeigneter, hier nicht weiter dargestellter, Mittel verbunden werden.

Das Einbring- oder Eindrehwerkzeug 36 enthält einen erfindungsgemäß im Bereich der axialen Stirnfläche 47 des Kopfteils 40 und / oder als kurze Stufe oder Ringbund ausgebildeten Anschlag 58 zur exakten Vorgabe einer definierten Eindrehtiefe des Implantats. Die Schiene 4 und insbesondere die Einbringhülse 54 enthalten einen weiteren Anschlag 60, mit welchem dem Anschlag 58 des Eindrehwerkzeugs 36 korrespondiert. Die Anschläge 58, 60, welche gemäß Fig. 5 bevorzugt im unteren bzw. dem Kiefer 2 zugewandten Endbereich der Schiene 4 und / oder innerhalb dieser liegen, begrenzen beim Eindrehen des Implantats 38 dessen Eindringtiefe in den Kiefer 2. Die Außengeometrie der Einbringhülse 54 ist an die Innengeometrie der ersten Führungshülse 8 angepasst. Der Innendurchmesser der Einbringhülse 54 bzw. der in dieser vorgesehenen Ausnehmung 52 ist im Wesentlichen gleich groß wie der Außendurchmesser des Kopfteils 40 des Eindrehwerkzeugs 36, und zwar abgesehen von dem weiteren radial nach innen gerichteten Steg 62 der Einbringhülse 54. In bevorzugter Weise befindet sich der weitere Anschlag 60 innerhalb der Schiene 4, so dass beim Erreichen der Endlage der weitere Anschlag 60 des Eindrehwerkzeugs 36 sich innerhalb der Schiene befindet. Im Rahmen der Erfindung kann alternativ zum Anschlag 60 das Eindrehwerkzeug 36 einen außerhalb bzw. oberhalb der Schiene 4 angeordneten Anschlag 60' aufweisen, welcher mit gestrichelten Linien angedeutet ist und welche zweckmäßig an der gemäß Zeichnung oberen Endfläche der Einbringhülse 54 bei Erreichen der maximalen Eindrehtiefe zur Anlage gelangt.

Weiterhin ist in Fig. 5 mittels gestrichelter Linie ein Miniaturimplantat 64 angedeutet, welches einen wesentlich kleineren Durchmesser als das Implantat 38 aufweist. Es sei hier davon ausgegangen, dass das dargestellte Implantat 38 dasjenige ist, welches in einem System von Implantaten mit unterschiedlichen Außendurchmessern den größten Außendurchmesser besitzt. Im Rahmen der Erfindung ist für sämtliche Implantate eines Implantatsystems, einschließlich Miniaturimplantaten, mit Außendurchmesser gleich oder kleiner als 1 mm, das gleiche und/oder insbesondere hinsichtlich der Ausbildung des Kopfteils 40 übereinstimmendes Eindrehwerkzeuge 36 vorgesehen, wodurch vor allem der Aufwand für die Herstellung und Bereitstellung des oder der Eindrehwerkzeuge erheblich reduziert ist. Darüber hinaus hat es sich als besonders vorteilhaft erwiesen, die Einbringhülse 54 übereinstimmend mit der Bohrhülse, insbesondere für das Implantat mit dem größten Durchmesser des lmplantatsystems auszubilden, so dass diese Bohrhülse gleichzeitig auch als Einbringhülse zum Einsatz gelangt. Durch das erfindungsgemäße Implantatsystem ist eine erhebliche Vereinfachung und Reduzierung der Bestandteile des Implantatsatzes samt zugehörender Hilfsmittel, nämlich der genannten Hülsen erreicht.

In einer besonderen Ausgestaltung der Erfindung können die erste Hülse und / oder die Bohrhülsen entfallen, wobei das Implantat als transgingivales Implantat unmittelbar und ohne Bohren oder Vorbereiten eines Implantatbetts in den Kiefer bzw. Kieferknochen eingebracht wird. Diese Ausgestaltung ist besonders für Miniimplantate vorgesehen, deren Außendurchmesser in der Größenordnung von 1 mm und ggf. auch kleiner vorgegeben sind. Auch bei derartigen transgingivalen und / oder einteiligen Implantaten geringen Durchmessers weist die Anordnung zum einen das Eindrehwerkzeug mit einer Anschlagfläche und ferner das Eindrehwerkzeug eine mit letzterer korrespondierenden Anschlagfläche zur Vorgabe einer exakten definierten Einbringtiefe des Implantats auf.

Fig. 6 zeigt nach Abnahme der Schiene das vollständig in den Kiefer 2 implantierte Implantat 38. Das einteilige Implantat 38 ist transgingival insertiert, wobei mittels der strichpunktierten 34 der wiederum Übergangsbereich zwischen Schleimhaut und Kieferknochen angedeutet ist.

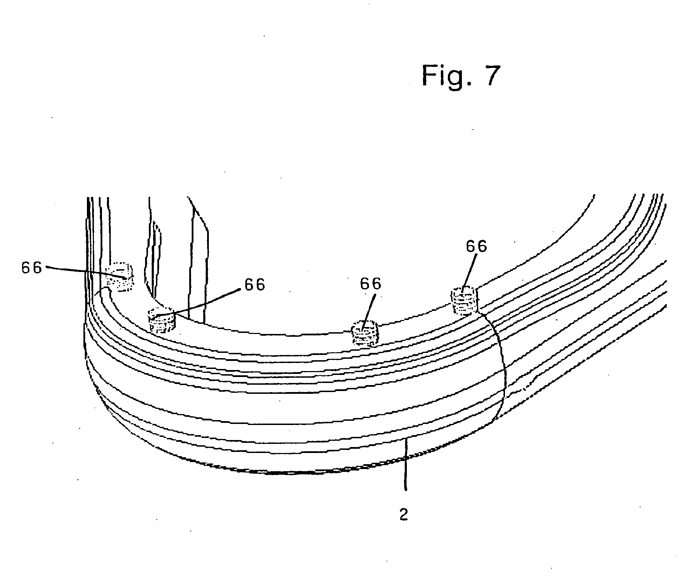

Fig. 7 zeigt schematisch den Kiefer 2 nach der Insertion von vier Implantaten. Die Implantate sind entsprechend Fig. 6 vollständig in den Kiefer insertiert, und es sind hier lediglich noch die mit den Implantaten verbundenen Verankerungskörper 66 für Kronen, Brücken oder dergleichen zu erkennen.

### Bezugszeichen

- 2: Kiefer
- 4: Schiene
- 6: Bohrung / Ausnehmung
- 8: Führungshülse
- 10: Bohrhülse
- 12: erstes Positionierungselement von 8
- 14: zweites Positionierungselement von 10
- 16: Längsachse
- 18: Anschlagfläche von 10
- 20: obere Endfläche von 10
- 22: Bohrer
- 24: Bohrwerkzeug
- 26: Anlagefläche von 24
- 28: Anlagefläche von 10
- 30: Steg in 10
- 32: Bohrung / Implantatbett
- 34: strichpunktierte Linie
- 36: Eindrehwerkzeug
- 38: Implantat
- 40: Kopfteil von 36
- 42: Schaft von 36
- 44: Durchmesser von 42
- 45: axiale Stirnfläche von 38
- 46: Länge von 36
- 47: axiale Stirnfläche von 40
- 48: Außenfläche von 40
- 50: Außendurchmesser von 40
- 52: Ausnehmung / Führungsfläche
- 54: Einbringhülse
- 56: Dicke von 4
- 58: Anschlag von 36
- 60: weiterer Anschlag von 4
- 62: Steg in 54
- 64: gestrichelte Linie / Miniaturimplantat
- 66: Verankerungskörper

## Patentansprüche

1. Anordnung zur Insertion von Implantaten (38), insbesondere Dentalimplantaten, enthaltend eine mit einer Bohrung (6) versehene Schiene (4), welche zur Positionierung des Implantats (38) auf einen für die Insertion vorgesehenen Bereich, insbesondere eines Knochens, ausgebildet und auf diesem anordenbar ist, und ferner enthaltend ein Eindrehwerkzeug (36) für das Implantat (38), wobei die Schiene (4) eine Führungsfläche (52) enthält, wobei das Eindrehwerkzeug (36) einen Kopfteil (40) aufweist, mit welchem das Implantat (38) verbunden ist und welcher eine mit der Führungsfläche (52) in Eingriff bringbare Außenfläche (48) aufweist, wobei das Eindrehwerkzeug (36) einen ersten Anschlag (58) aufweist und wobei die Schiene (4) einen weiteren Anschlag (60) zumindest mittelbar enthält oder aufweist, an welchem der erste Anschlag (58) des Eindrehwerkzeugs (36) beim Eindrehen des Implantats (38) zur Vorgabe dessen Einbringtiefe zur Anlage gelangt.
**dadurch gekennzeichnet, dass** eine in der Bohrung (6) anordenbare Einbringhülse (54) für das Implantat (38) vorgesehen ist, welche die Führungsfläche (52) für den Kopfteil (40) des Eindrehwerkzeugs (36) und ferner den weiteren Anschlag (60) enthält,
dass für Implantate (38) mit unterschiedlichen Außendurchmessern die Einbringhülsen (54) übereinstimmend ausgebildete Führungsflächen (52) aufweisen
und dass die Schiene (4) einerseits und die Einbringhülse (54) ebenso wie eine in der Bohrung (6) anordenbare Bohrhülse (10) andererseits miteinander korrespondierende Positionierungselemente (12, 14) für die axiale Positionierung bezüglich einer Längsachse (16) der Bohrung (6) in der Schiene (4) aufweisen.

2. Anordnung nach Anspruch 1, **dadurch gekennzeichnet, dass** der weitere Anschlag (60) in einem dem Kiefer zugewandten Endbereich der Einbringhülse (54) angeordnet ist.

3. Anordnung nach Anspruch 1 oder 2, **dadurch gekennzeichnet, dass** der Kopfteil (40) des Eindrehwerkzeugs (36) den ersten Anschlag (58) aufweist und/oder dass der Kopfteil (40) eine axiale Länge (46) aufweist, welche zumindest gleich groß ist wie die Dicke (56) der Schiene (4) oder bevorzugt um einen vorgegebenen Faktor größer als die Dicke (56) ist, wobei der Faktor maximal vorzugsweise den Wert 2 aufweist und insbesondere maximal den Wert 1,5 aufweist.

4. Anordnung nach Anspruch 2 oder 3, **dadurch gekennzeichnet, dass** eine vorgegebene Anzahl von Einbringhülsen (54) vorgesehen ist, welche die übereinstimmend ausgebildete Führungsflächen (52) aufweisen und insgesamt übereinstimmend ausgebildet sind.

5. Anordnung nach einem der Ansprüche 1 bis 4, **dadurch gekennzeichnet, dass** der Kopfteil (40) mit einer axialen Stirnfläche (45) unmittelbar an einer axialen Endfläche (39) des Implantats (38) anliegt und / oder dass an den Kopfteil (40) ein Schaft (42) unmittelbar anschließend angeordnet ist, dessen Durchmesser wesentlich kleiner ist als der Durchmesser (50) des Kopfteils (40).

6. Anordnung nach einem der Ansprüche 1 bis 5, **dadurch gekennzeichnet, dass** die Schiene (4) eine zumindest teilweise innerhalb der Bohrung (6) angeordnete Führungshülse (8) enthält und / oder dass die Führungshülse (8) mit der Schiene (4) fest oder lösbar verbunden ist.

7. Anordnung nach einem der Ansprüche 1 bis 6, **dadurch gekennzeichnet, dass** die in der Bohrung (6) und/oder der Führungshülse (8) anordenbare Bohrhülse (10) eine an die Außenkontur eines Bohrers (22) angepasste Innenkontur aufweist.

8. Anordnung nach einem der Ansprüche 1 bis 7 **dadurch gekennzeichnet, dass** die Anordnung zur transgingivalen Einbringung des Implantats (38) vorgesehen ist und/ oder dass das Implantat (38) als ein Miniaturimplantat ausgebildet ist.

9. Anordnung nach einem der Ansprüche 1 bis 8, **dadurch gekennzeichnet, dass** der weitere Anschlag (60) an einem radial nach innen gerichteten Steg (62) innerhalb der Einbringhülse (54) angeordnet ist oder dass der weitere Anschlag (60) durch eine insbesondere obere Endfläche im Bereich der Einbringhülse (54) gebildet ist.

## Claims

1. An arrangement for inserting implants (38), in particular dental implants, including a template (4), which is provided with a bore (6) and which is embodied for positioning the implant (38) on an area, which is provided for the insertion, in particular a bone, and which can be arranged thereon, and further including a screw-in tool (36) for the implant (38), wherein the template (4) includes a guide surface (52), wherein the screw-in tool (36) encompasses a head part (40), to which the implant (38) is connected and which encompasses an outer surface (48), which can be engaged with the guide surface (52), wherein the screw-in tool (36) encompasses a first stop (58) and wherein the template (4) includes or encompasses a further stop (60) at least indirectly, on which the first stop (58) of the screw-in tool (36) comes to rest to specify the insertion depth of the implant (38) when it is being screwed in,
**characterized in that** an insertion bushing (54), which can be arranged in the bore (6) and which includes the guide surface (52) for the head part (40) of the screw-in tool (36) and further the additional stop (60) is provided for the implant (38),
that the insertion bushings (54) for implants (38) comprising different outer diameters encompass guide surfaces (52), which are embodied so as to correspond, and that the template (4) on the one hand and the insertion bushing (54) as well as a bore bushing (10), which can be arranged in the bore (6) on the other hand, encompass positioning elements (12, 14), which correspond to one another, for the axial positioning with reference to a longitudinal axis (16) of the bore (6) in the template (4).

2. The arrangement according to claim 1, **characterized in that** the further stop (60) is arranged in an end area of the insertion bushing (54), which faces the jaw.

3. The arrangement according to claim 1 or 2, **characterized in that** the head part (40) of the screw-in tool (36) encompasses the first stop (58) and/or that the head part (40) encompasses an axial length (46), which has at least the same size as the thickness (56) of the template (4) or is preferably greater than the thickness (56) by a predetermined factor, wherein the factor preferably encompasses at most the value 2 and in particular encompasses at most the value 1.5.

4. The arrangement according to claim 2 or 3, **characterized in that** provision is made for a predetermined number of insertion bushings (54), which encompass guide surfaces (52), which are embodied so as to correspond, and which are embodied so as to correspond as a whole.

5. The arrangement according to one of claims 1 to 4, **characterized in that** the head part (40) with an axial front face (45) rests directly on an axial end surface (39) of the implant (38) and/or that a shank (42) is arranged so as to directly follow the head part (40), the diameter of which is considerably smaller than the diameter (50) of the head part (40).

6. The arrangement according to one of claims 1 to 5, **characterized in that** the template (4) includes a guide bushing (8), which is at least partially arranged within the bore (6) and/or that the guide bushing (8) is connected to the template (4) in a fixed manner or so as to be capable of being removed.

7. The arrangement according to one of claims 1 to 6, **characterized in that** the bore bushing (10), which can be arranged in the bore (6) and/or in the guide bushing (8), encompasses an inner contour, which is adapted to the outer contour of a drill (22).

8. The arrangement according to one of claims 1 to 7, **characterized in that** provision is made for the arrangement for the transgingival insertion of the implant (38) and/or **in that** the implant (38) is embodied as a miniature implant.

9. The arrangement according to one of claims 1 to 8, **characterized in that** the further stop (60) is arranged at a bar (62), which is oriented radially inwardly, within the insertion bushing (54), or **in that** the further stop (60) is formed by means of an in particular upper end surface in the area of the insertion bushing (54).

## Revendications

1. Système d'insertion d'implants (38), en particulier d'implants dentaires, comprenant un rail (4) muni d'un perçage (6) et réalisé pour positionner l'implant (38) sur une zone, en particulier d'un os, prévue pour l'insertion, et pouvant être disposé sur celle-ci, et comprenant en outre un outil de vissage (36) pour l'implant (38), dans lequel le rail (4) comprend une surface de guidage (52), dans lequel l'outil de vissage (36) présente une partie de tête (40) à laquelle est relié l'implant (38) et qui présente une surface extérieure (48) pouvant être mise en prise avec la surface de guidage (52), dans lequel l'outil de vissage (36) présente une première butée (58), et dans lequel le rail (4) comprend ou présente au moins indirectement une butée supplémentaire (60) sur laquelle s'appuie la première butée (58) de l'outil de vissage (36) lors du vissage de l'implant (38) pour spécifier la profondeur d'insertion de celui-ci,
**caractérisé en ce qu'**il est prévu une douille d'insertion (54) pour l'implant (38), qui peut être disposée dans le perçage (6) et qui comprend la surface de guidage (52) pour la partie de tête (40) de l'outil de vissage (36) et en outre la butée supplémentaire (60),
**en ce que** pour des implants (38) de différents diamètres extérieurs, les douilles d'insertion (54) présentent des surfaces de guidage (54) réalisées de façon concordante,
et **en ce que** le rail d'une part et la douille d'insertion (54) ainsi qu'une douille de perçage (10) pouvant être disposée dans le perçage (6) d'autre part présentent des éléments de positionnement (12, 14) correspondant l'un à l'autre pour le positionnement axial par rapport à un axe longitudinal (16) du perçage (6) dans le rail (4).

2. Système selon la revendication 1, **caractérisé en ce que** la butée supplémentaire (60) est disposée dans une zone d'extrémité de la douille d'insertion (54) tournée vers la mâchoire.

3. Système selon la revendication 1 ou 2, **caractérisé en ce que** la partie de tête (40) de l'outil de vissage (36) présente la première butée (58) et/ou **en ce que** la partie de tête (40) présente une longueur axiale (46) qui est au moins aussi grande que l'épaisseur (56) du rail (4) ou de préférence plus grande que l'épaisseur (56) d'un facteur spécifié, ledit facteur présentant au maximum de préférence la valeur 2 et en particulier au maximum la valeur 1,5.

4. Système selon la revendication 2 ou 3, **caractérisé en ce qu'**un nombre spécifié de douilles d'insertion (54) est prévu qui présentent des surfaces de guidage (52) réalisées de façon concordante et qui sont réalisées globalement de façon concordante.

5. Système selon l'une quelconque des revendications 1 à 4, **caractérisé en ce que** la partie de tête (40) s'applique par une surface frontale axiale (45) directement contre une surface d'extrémité axiale (39) de l'implant (38) et/ou **en ce qu'**une tige (42) est disposée de façon directement adjacente à la partie de tête (40) et dont le diamètre est nettement inférieur au diamètre (50) de la partie de tête (40).

6. Système selon l'une quelconque des revendications 1 à 5, **caractérisé en ce que** le rail (4) comprend une douille de guidage (8) disposée au moins partiellement à l'intérieur du perçage (6) et/ou **en ce que** la douille de guidage (8) est reliée au rail (4) de façon fixe ou amovible.

7. Système selon l'une quelconque des revendications 1 à 6, **caractérisé en ce que** la douille de perçage (10) qui peut être disposée dans le perçage (6) et/ou la douille de guidage (8) présente un contour intérieur adapté au contour extérieur d'une fraise (22).

8. Système selon l'une quelconque des revendications 1 à 7, **caractérisé en ce que** le système est prévu pour l'insertion transgingivale de l'implant (38) et/ou **en ce que** l'implant (38) est réalisé comme un implant miniature.

9. Système selon l'une quelconque des revendications 1 à 8, **caractérisé en ce que** la butée supplémentaire (60) est disposée à l'intérieur de la douille d'insertion (54) au niveau d'une barrette (62) tournée radialement vers l'intérieur, ou **en ce que** la butée supplémentaire (60) est réalisée par une surface d'extrémité, en particulier supérieure, au niveau de la douille d'insertion (54).
